# EUROPEAN PATENT APPLICATION

(11) **EP 1 693 669 A2**
(43) Date of publication of application: **23.08.2006**
(21) Application number: 06002907.1
(22) Date of filing: 14.02.2006
(51) Int. Cl.: G01N 33/00

(54) **Device for measuring concentrations of carbon dioxide and oxygen**

(30) Priority: 17.02.2005 IT MI20050232
(71) Applicant: Finanziaria Unterland S.p.A., 39100 Bolzano (IT)
(72) Inventor: Pruneri, Marco, 39040 Ora (Bolzano) (IT)
(74) Representative: Forattini, Amelia

(57) **Abstract**

A device for measuring the concentrations of carbon dioxide and oxygen, comprising a sensor module which is functionally connected to an analysis card connected to a user interface. The module comprises carbon dioxide and oxygen sensors and a means for compensating temperature and humidity variations so that the reading of the sensors is corrected as a function of the temperature and humidity variations of the mixture, in order to obtain measurements which are independent of the boundary conditions.

## Description

The present invention relates to a device for measuring concentrations of carbon dioxide and oxygen in the atmosphere.

So-called "thermostatically-controlled" O₂ and CO₂ sensors are known and currently commercially available. In such devices the gas sensor is kept at a preset temperature so as to provide a faithful indication of the detected gas level.

However, conventional thermostatically-controlled systems have drawbacks.

A first drawback is due to the fact that variations in the humidity of the mixture introduce errors that cannot be compensated by thermostatically-controlled systems.

Thermostatically-controlled sensors that use an ordinary resistor to heat the sensor have the drawback that any rise in the temperature of the input mixture introduces an error which cannot be compensated.

Also, the thermostat is penalized by a more or less substantial inertia, which allows it to correct the variations of the boundary conditions, specifically the temperature variations, only after a certain settling period thereof; this means that the measurement is affected by an error which sometimes is very substantial during transient periods.

The module with thermostatically-controlled sensor necessarily consumes considerable power and is scarcely suitable to be used in portable battery-powered applications.

Thermostatically-controlled modules are usually pure analog devices and are therefore characterized by a limited capacity to compensate the boundary variations of analyses and by limited linearity.

The aim of the present invention is to provide a device for measuring the concentrations of carbon dioxide and oxygen which overcomes the drawbacks of the cited prior art.

An object of the invention is to provide a measurement device which is capable of giving an accurate reading even in the presence of variations in humidity and of high temperatures of the input mixture.

A further object of the invention is to provide a measurement device which is not penalized by operating inertia, differently from conventional analyzers, in which the measurement can be affected by a sometimes very substantial error during transient periods.

A further object is to provide a measurement device which has a reduced energy consumption.

This aim and these and other objects, which will become better apparent hereinafter, are achieved by a device for measuring the concentrations of carbon dioxide and oxygen, characterized in that it comprises a sensor module which is functionally connected to an analysis card which in turn is connected to a user interface, said module comprising carbon dioxide and oxygen sensors and a means for compensating temperature and humidity variations so that the reading of the sensors is corrected as a function of the temperature and humidity variations of the mixture, in order to obtain measurements that are independent of the boundary conditions.

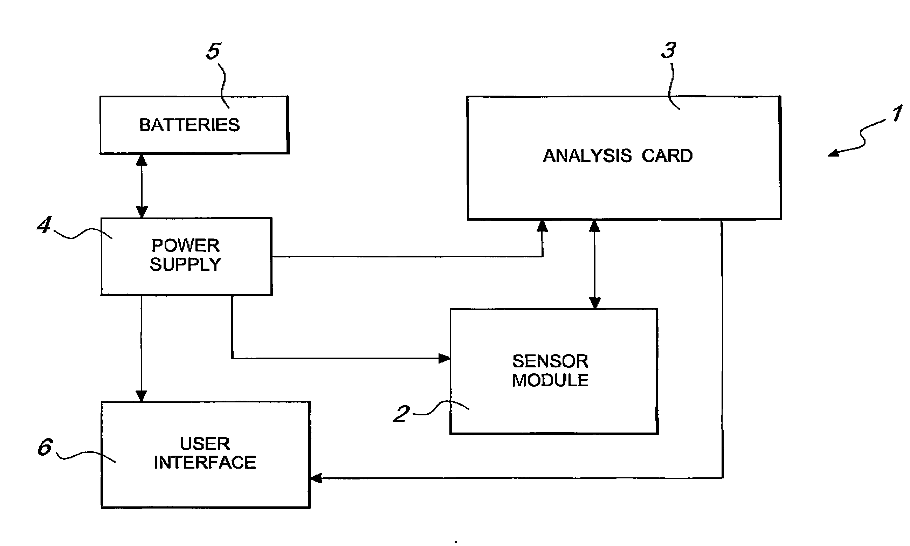

Further characteristics and advantages of the invention will become better apparent from the following detailed description of preferred but not exclusive embodiments thereof, illustrated by way of non-limiting example in the accompanying drawing, wherein the only figure is a block diagram of an analyzer provided with a measurement device according to the invention.

With reference to the figure, the device for measuring concentrations of carbon dioxide and oxygen comprises a sensor module 2 which is functionally connected to an analysis card 3.

By way of example the measurement device according to the invention is shown applied to a portable gas analyzer, generally designated by the reference numeral 1.

The instrument 1 can be powered by the electrical mains at 220 V (50 Hz) and has an auxiliary power supply 4, provided with batteries 5 which, because of its extremely low energy consumption, ensures a long operating time.

The analysis card 3, which includes digital electronics provided with a powerful microprocessor, processes the signals that arrive from the sensor module 2 and manages the user interface 6.

Measurement of the carbon dioxide is performed by means of an infrared sensor, while measurement of the oxygen occurs by means of an electrochemical cell.

Both measurements are extremely accurate and precise by virtue of the system which ensures compensation of temperature variations, provided within the analysis sensor module.

The sensor module has been designed specifically to be integrated easily in a wide range of systems and applications that require high performance and accuracy in the measurement of concentrations of O₂ and CO₂ gases, using components of high quality and reliability.

The module comprises a microcontroller and an analog/digital converter with very high resolution (16-bit) for sampling the signals of the sensors.

The amplifier stage used in the module, according to the present invention, is characterized by extremely low noise and by excellent temperature stability.

The reading of the sensors is corrected as a function of the temperature and humidity variations of the mixture in order to obtain measurements which are independent of the boundary conditions.

Temperature and humidity compensation is performed by using two complementary sensors, a temperature sensor and a relative humidity sensor. The readings of those sensors are used in the algorithms for correcting the measurement of the oxygen and carbon dioxide on the part of the microcontroller on board the sensor module.

The components, in SMD technology, ensure maximum reliability.

CO₂ analysis is performed by means of an infrared sensor which is temperature-compensated and is driven by very low-noise and low-consumption electronics of the latest generation.

Oxygen analysis is assigned to an optional electrochemical oxygen cell.

The analyzer according to the invention can be provided alternately with a microsensor or with a long-life sensor, according to the specific requirements of the user.

The microsensor, which lasts three years, allows to keep the profile of the analysis module within 35 mm of height, allowing its mounting even in very compact portable analyzers.

The long-life sensor, with a life of approximately ten years, is larger and is more suitable for mounting in instruments wherein the dimensions are not a particularly important constraint but a very long operating life is required.

The apparatus is provided with analog outputs that have an interval which can be programmed by means of parameters that set the scale start and full-scale values. Those operations can be performed in the factory.

Preferably, the output is a 0-20 mA current or a 0-1 V voltage if the appropriately provided jumper is inserted. In this case, the connected user device is preferably at a high impedance in order to avoid biasing the measurement.

On request, it is possible to provide the sensor module with outputs of different values, for example 4-20 mA.

The module also contains a serial interface (of the RS-485 type) for transmitting the measured data.

A half duplex protocol accepts one master and one or more slaves (a maximum of 31) and uses the binary method (RTU) at 9600 baud: 1 start bit, 8 data bits, 1 EVEN parity bit, 1 STOP bit.

The module is completely tested and calibrated, preferably in specialized laboratories, but it is in any case possible to connect potentiometers externally thereto in order to introduce a compensation on the analog outputs.

For example, a compensation equal to +/- 20% of the full-scale value is possible.

An offset equal to the value defined above is introduced on the ZERO setting, while a gain is introduced in the SPAN setting which provides the above cited variation on the full-scale value.

The analyzer provided with the sensor module according to the invention therefore offers great reliability, high precision, measurement stability which is independent of variations in temperature and relative humidity of the mixture to be analyzed, low consumption and compact dimensions.

In practice it has been found that the invention achieves the intended aim and objects, a device having been provided in which the use of a double complementary sensor, or temperature and relative humidity, to compensate the analyses, allows to provide a device which is qualitatively superior to the best thermostatically-controfled sensors currently commercially available, in which the variations of the humidity of the mixture introduce errors that cannot be compensated.

The thermostat of a traditional analyzer, and in general any sensor which does not use complementary temperature and humidity sensors, together with calculation methods for compensating the boundary conditions of the analyses, is penalized by a more or less substantial inertia, which allows it to correct the variations in boundary conditions, specifically temperature variations, only after a certain settling period thereof. This means that the measurement of a traditional sensor is affected by an error which is sometimes very large during transient periods, differently from the device according to the present invention.

Also, a traditional thermostatically-controlled sensor necessarily uses much more power than the device according to the present invention, which uses complementary sensors and numerical methods to compensate for the boundary conditions.

Because of its limited energy consumption, the device according to the present invention is particularly advantageous in portable battery-powered applications.

Differently from traditional thermostatically-controlled sensors, which are purely analog and are characterized by a limited capacity to compensate for variations in the boundary conditions of the analyses, and by a lower linearity, the device according to the present invention is provided with a microcontroller which allows much more extreme and faster linearization and compensation operations.

The device according to the invention is susceptible of numerous modifications and variations, within the scope of the appended claims. All the details may be replaced with technically equivalent elements.

The materials used, as well as the dimensions, may of course be any according to requirements and to the state of the art.

## Claims

1. A device for measuring the concentrations of carbon dioxide and oxygen, **characterized in that** it comprises a sensor module which is functionally connected to an analysis card connected to a user interface card, said module comprising carbon dioxide and oxygen sensors and a means for compensating temperature and humidity variations so that the reading of the sensors is corrected as a function of the temperature and humidity variations of the mixture, in order to obtain measurements that are independent of the boundary conditions.

2. The measurement device according to claim 1, **characterized in that** said module comprises digital electronics provided with a microprocessor which processes the signals that arrive from said sensors.

3. The measurement device according to claim 1 or 2, **characterized in that** it comprises an infrared sensor for measuring carbon dioxide.

4. The measurement device according to one or more of the preceding claims, **characterized in that** it comprises an electrochemical cell for measuring oxygen.

5. The measurement device according to one or more of the preceding claims, **characterized in that** said module comprises a microprocessor and a very high resolution (16-bit) analog/digital converter for sampling the signals of the sensors.

6. The measurement device according to one or more of the preceding claims, **characterized in that** said module comprises an amplifier stage **characterized by** very low noise and high thermal stability.

7. The measurement device according to one or more of the preceding claims, **characterized in that** said temperature and moisture compensation means comprises two complementary sensors, a temperature sensor and a relative humidity sensor, the measurements of said sensors being processed by said microcontroller and being used in the algorithms for correcting the oxygen and carbon dioxide measurement.

8. The measurement device according to one or more of the preceding claims,
**characterized in that** it comprises components in SMD technology.

9. The measurement device according to one or more of the preceding claims, **characterized in that** said infrared sensor for analyzing CO₂ is a temperature-compensated infrared sensor driven by very low-noise and low-power electronics.

10. The measurement device according to one or more of the preceding claims,
**characterized in that** it comprises an oxygen microsensor.

11. The measurement device according to one or more of the preceding claims, **characterized in that** it comprises a long-life oxygen sensor.

12. The measurement device according to one or more of the preceding claims, **characterized in that** said microsensor has a life of approximately three years and is compact, for installation in compact analyzers.

13. The measurement device according to one or more of the preceding claims, **characterized in that** said long-life duration sensor, with a life of approximately ten years, is larger and is suitable for mounting in larger instruments which however have a long analysis life.

14. The measurement device according to one or more of the preceding claims, **characterized in that** it comprises analog outputs which have a range which can be programmed by means of parameters which set the scale start and full-scale values.

15. The measurement device according to one or more of the preceding claims, **characterized in that** said output is a current of 0-20 mA in current or a voltage of 0-1 V.

16. The measurement device according to claim 15, **characterized in that** the connected user device has a high impedance.

17. The measurement device according to one or more of the preceding claims, **characterized in that** said output is a current of 0-20 mA.

18. The measurement device according to one or more of the preceding claims, **characterized in that** said output is a current of 4-20 mA.

19. The measurement device according to one or more of the preceding claims, **characterized in that** said module comprises a serial interface (of the RS485 type) for transmitting the measured data.

20. The measurement device according to one or more of the preceding claims, **characterized in that** it comprises a haff-duplex protocol which accepts one master and one or more slaves and uses the binary method (RTU) at 9600 baud: 1 start bit, 8 data bits, 1 EVEN parity bit, 1 STOP bit.

21. The measurement device according to one or more of the preceding claims, **characterized in that** the compensation is equal to +/-20% of the full-scale value.

22. A portable gas analyzer, particularly for measuring concentrations of carbon dioxide and oxygen, **characterized in that** it comprises a sensor module which is functionally connected to an analysis card, which in turn is connected to a user interface card, said module comprising carbon dioxide and oxygen sensors and a means for compensating temperature and humidity variations so that the reading of the sensors is corrected according to the temperature and humidity variations of the mixture, in order to obtain measurements which are independent of the boundary conditions.

23. The gas analyzer according to claim 22, **characterized in that** it is supplied by the electric mains and comprises an auxiliary battery-powered supply.
